# EUROPEAN PATENT SPECIFICATION

(11) **EP 2 274 267 B1**
(45) Date of publication and mention of the grant of the patent: **04.01.2012**
(21) Application number: 09728835.1
(22) Date of filing: 31.03.2009
(51) Int. Cl.: C07C 67/58, C07C 69/33

(54) **PRAVASTATIN EXTRACTION**
EXTRAKTION VON PRAVASTATIN
EXTRACTION DE PRAVASTATINE

(30) Priority: 02.04.2008 EP 08103319
(43) Date of publication of application: 19.01.2011
(73) Proprietor: DSM IP Assets B.V., 6411 TE Heerlen (NL)
(72) Inventor: Bouman, Aad Johannes, NL-3077 PL Rotterdam (NL); Pater De, , Robertus Mattheus, NL-2613 ZL Delft (NL); Wnukowski, Piotr, NL-2668 CE Delft (NL)
(74) Representative: Cazemier, Anne Engeline
(86) International application number: PCT/EP2009/053791
(87) International publication number: WO 2009/121870

(56) References cited:
- EP-A- 0 877 089
- WO-A-00/46175
- WO-A-01/39768
- WO-A-2007/147827
- US-A1- 2003 199 047
- US-A1- 2004 138 294

## Description

### Field of the invention

The present invention relates to the extraction of the HMG-CoA reductase inhibitor pravastatin and the concomitant removal of impurities.

### Background of the invention

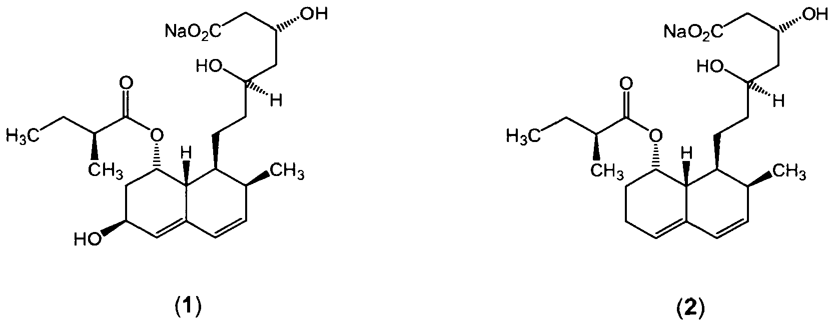

Pravastatin is an inhibitor of the enzyme (3*S*)-hydroxy-3-methylglutarylcoenzyme A (HMG-CoA) reductase. The sodium salt of pravastatin, pravastatin sodium (**1**) is a potent cholesterol-lowering agent which is commercially available for the treatment of hyperlipidemia. Pravastatin can be prepared by microbial oxidation of the fermentatively obtained precursor compactin, the sodium salt of which has structure (**2**), such as described for instance in US 4,346,227. Recently, an improved approach has been described in WO 2007/147827 in which pravastatin is prepared directly in a host cell equipped with genes for compactin biosynthesis and a gene for conversion of compactin into pravastatin.

Despite the progress made thus far in pravastatin production processes, there still remain disadvantages that need to be solved. One such disadvantage of the prior art methods is that traces of compactin (**2**), either as free acid, as ionized salt or in the lactone form can still be present in the end product. As a result of the fact that this impurity is structurally closely related to pravastatin, its removal from pravastatin samples is tedious.

As the purity of an active pharmaceutical intermediate is important for the production of a safe and effective medicament, purification of pravastatin has often been addressed in the prior art.

In WO 92/16276 a method is described which uses high performance liquid chromatography (HPLC). Although pravastatin with purity larger than 99.5% is mentioned, there is no mention as to how this methodology relates to compactin as impurity. Moreover, HPLC has several disadvantages, namely the need for recycling of resin, the use of excessive amounts of solvents and the poor applicability on industrial scale. As a matter of fact, the large-scale purification of pravastatin is not disclosed in WO 92/16276.

In WO 99/42601 an isolation/purification process is disclosed wherein an aqueous pravastatin solution is acidified and subsequently extracted with ethyl acetate after which several crystallization steps are carried out. Although this procedure is disclosed at relatively large scale (starting from 30 liters of a fermentation broth) there is no indication towards the applicability of the method for purification of pravastatin containing compactin.

In WO 00/17182 a purification process is described based on displacement chromatography. Described is the purification of lab-scale (i.e. 1 gram and smaller) samples of pravastatin by displacement chromatography. Although purities of pravastatin sodium of 99.7% and 99.8% are mentioned, these purities refer to pravastatin in solution. Isolated pravastatin sodium is not disclosed, nor is there any indication to the effect of the method on the removal of compactin.

Patent application US 2003/0216596 describes decomposition of impurities with inorganic acids (such as HBr, HCl, H₂SO₄ HClO₄, H₃PO₄ and HNO₃) or bases (such as alkali metal carbonates, bicarbonates, hydrides, hydroxides and alkoxides). Disclosed is purified isolated pravastatin sodium at semi large scale (*i.e*. up to 32 gram samples) having purities up to 99.67% and the application focuses on the removal of 6-epi-pravastatin and is silent with respect to compactin nor gives a suggestion as to whether the method would be suitable to reduce the presence of compactin.

Still further isolation/purification processes have been disclosed in EP 877089, US 2003/199047, US 2004/138294, WO 00/46175 and WO 01/39768 although neither of these methods mentions an efficient reduction of the amounts of compactin in the end product pravastatin.

Consequently, there is room for a still further improved purification method for pravastatin, more specifically with respect to the presence of the impurity compactin.

### Detailed description of the invention

The first aspect of the present invention is to provide a method for the purification of pravastatin or a pharmacologically acceptable salt thereof. It has been found that extraction of a solution containing pravastatin and compactin in a water-immiscible solvent with water or an aqueous solution at a pH value ranging from 5.0 to 6.5 results in an aqueous solution in which the ratio pravastatin:compactin has been increased. Extraction of pravastatin from an organic into an aqueous phase at a pH value ranging from 5.0 to 6.5 is unprecedented as prior art advocates to perform such extractions above pH 7.0 where distribution coefficients are favorable, as would be in line with extractions of other organic acids. Moreover, on the basis of the high similarity between the molecular structures of pravastatin and compactin a significant difference in distribution coefficients would not be expected beforehand. However, it was found that using the method of the present invention, the ratio pravastatin:compactin could be improved from 3:1 in the starting mixture to up to 100:1 after extraction.

In the context of the present invention, a water-immiscible solvent is a solvent of which the solubility in water at 20±2°C is lower than 15%, preferably lower than 10%, more preferably lower than 8%, most preferably between 0.5% and 5%. Specific examples of water-immiscible solvents that may be utilized in the present invention include water-immiscible esters, such as ethyl acetate, isopropyl acetate, methyl acetate, *n*-propyl acetate, isobutyl acetate, *n*-butyl acetate, isobutyl isobutyrate, 2-ethylhexyl acetate, ethylene glycol diacetate, water-immiscible ketones such as methyl ethyl ketone, methyl isobutyl ketone, methyl isoamyl ketone, methyl *n*-amyl ketone, diisobutyl ketone, cyclohexanone and isophorone, water-immiscible ether esters such as ethyl 3-ethoxypropionate, water-immiscible aromatic hydrocarbons such as toluene and xylene, water-immiscible halohydrocarbons such as chloroform, dichloromethane, 1,1,1-trichloroethane and water-immiscible glycol ether esters such as propylene glycol monomethyl ether acetate, ethylene glycol monoethyl ether acetate, ethylene glycol monobutyl ether acetate and diethylene glycol monobutyl ether acetate.

The pH-range for extraction of pravastatin from an organic into an aqueous phase is from 5.5 to 6.5, most preferably from 5.7 to 6.0. Achievement of the desired pH value can be done using acids and bases that are known to the skilled person and/or known to be suitable for extracting pravastatin, such as for instance bases like ammonium hydroxide, potassium hydroxide, sodium hydroxide and the like and, if needed, acids such as HBr, HCl, H₂SO₄ HClO₄, H₃PO₄, HNO₃ and the like.

In one embodiment, the method of the present invention is particularly useful for purifying pravastatin as obtained through biotechnological processes, for instance fermentation of which alternative approaches are known. In one approach, pravastatin is produced via two sequential fermentations. First *Penicillium citrinum* produces compactin, of which the lactone ring is chemically hydrolyzed with sodium hydroxide. Subsequently this is fed to a cultivation of *Streptomyces carbophilus,* which hydroxylates it to pravastatin (Metkinen News March 2000, Metkinen Oy, Finland; reviewed by Manzoni and Rollini, 2002, Appl. Microbiol. Biotechnol. 58:555-564). In another approach, also a one-step fermentation process for pravastatin in a *Penicillium chrysogenum* transformant has been disclosed (WO 2007/147827). Hence, according to the present invention, a solution of pravastatin in a water-immiscible solvent can be obtained by extracting an aqueous fermentation broth, which can for instance be obtained according to any of the fermentation processes described above, with said water-immiscible solvent, preferably at low pH values such as from 1 to 5, preferably from 2 to 4.5, more preferably from 3 to 4. Optionally cell materials and other solids are removed prior to extraction. The solution of pravastatin in a water-immiscible solvent thus obtained is then subjected to extraction with water or an aqueous solution according to the method of the present invention.

In another embodiment the extraction can be further optimized by carrying out said extraction in a counter-current or in a cross-current mode. This usually increases the extraction yield on pravastatin while keeping the ratio pravastatin:compactin at a good level. In one example, the ratio pravastatin:compactin was 3:1 in the starting mixture, 45:1 after one back-extraction, 25:1 after two back-extractions and 17:1 after three back-extractions where overall extraction yields of up to 99% could be achieved. When starting materials with ratio's of pravastatin:compactin higher than 3:1, for instance 5:1, 10:1 or 20:1 are used, a single back-extraction into the aqueous phase can give aqueous solutions with ratio's of pravastatin:compactin of 50:1, 100:1, 250:1, 500:1 or even 1000:1.

The second aspect of the invention discloses to a composition comprising pravastatin and compactin in which the ratio pravastatin:compactin is higher than 500:1 such as 600:1, 700:1, 800:1, 900:1, 1000:1 or intermediate values. Such a composition can be obtained according to the first aspect of the invention, i.e. by purifying a fermentatively prepared pravastatin broth by means of extraction into a water-immiscible solvent followed by back-extraction at a pH value ranging from 5.0 to 7.0 into an aqueous phase.

One embodiment of the second aspect of the invention discloses to the active pharmaceutical pravastatin itself or acceptable pharmaceutical salts thereof, notably the sodium salt. The method of the present invention hitherto is the first disclosure of successful removal of compactin from mixtures comprising both pravastatin and compactin. Furthermore, the method is suitable for use on an industrial scale. Therefore, industrial batches of pravastatin are obtainable by the method of the present invention. Hence, the present invention discloses a composition comprising pravastatin sodium and compactin the quantity of which is 0.2% or less by weight of the composition. Preferably the quantity of compactin is less than 0.1% by weight of the composition, more preferably less than 0.05% by weight of the composition. The assay of pravastatin sodium in the composition preferably is 99.6% or higher, more preferably 99.7% or higher, most preferably 99.8% or higher, still most preferably 99.9% or higher by weight of the composition.

In another embodiment, the present invention discloses pravastatin sodium of high purity on an industrial scale. In contrast with the prior art, the present invention suitably yields pravastatin sodium batches larger than 50 g, preferably of from 100 g to 10 tonnes, more preferably of from 500 g to 5 tonnes, most preferably of from 1 kg to 1000 kg, still most preferably of from 10 kg to 100 kg. The above pravastatin batches have only minor impurities as the assay of pravastatin sodium is 99.4% or higher, more preferably 99.6% or higher, most preferably 99.7% or higher, still most preferably 99.8% or higher by weight of the composition and the assay of compactin is 0.2% or lower, preferably 0.15% or lower, more preferably 0.10% or lower, most preferably 0.15% or lower by weight of the composition.

### EXAMPLES

### HPLC analysis

HPLC analysis was based on reversed phase liquid chromatography followed by UV-detection at 238 nm.
Apparatus: Dionex HPLC-UV system comprising of a P680 pump, a TCC-100 column compartment with thermostat, a WPS-3000 auto sampler and a UVD34OU PDA detector.
Conditions:

| | | |
|---|---|---|
| Column: | Waters Sunfire C18, 150* 4.6mm, 3.5 µm | |
| Column temp: | 40°C | |
| Flow rate: | 1.2 ml/min | |
| UV-detection | 238 nm | |
| Injection volume: | 10 µl | |
| Sample tray temp: | 5°C | |
| Mobile phase A: | 0.1% formic acid in methanol-Milli-Q purified water (6/4)v/v | |
| Mobile phase B: | 0.04% formic acid in acetonitrile | |
| Gradient: | T= 0 min. | 0 % B |
| | T= 5 min. | 0 % B |
| | T= 15 min. | 60 % B |
| | T= 16 min. | 60 % B |
| | T= 17 min. | 0 % B |
| | T= 20 min. | 0 % B |

Materials: Water: Milli-Q purified water or HPLC grade; acetonitrile, gradient grade, Merck art. Nr. 1.00030; methanol, gradient grade, Merck art. Nr. 1.06007; formic acid, Gr for analysis, Merck art. Nr.1.00264.
Procedures: Mobile phases:
- Mobile phase A: Mix 600ml methanol, 400ml MilliQ-purified water and 1 ml formic acid.
- Mobile phase B: Add 0.4 ml formic acid to 1 liter acetonitrile.

### pH measurement

pH values mentioned in the description and claims of the present application were measured using a Radiometer PHM82 standard pH meter equipped with a Mettler Toledo Inlab 412 pH electrode (electrolyte 9823). Equilibration was carried out at 20±1°C using a Merck pH 4.00 buffer (art. nr. 1.09435.1000) and a Merck pH 7.00 buffer (art. nr. 1.09439.1000).

### Example 1

### Separation between pravastatin and compactin by batch-wise single stage back-extraction from ethyl acetate

A broth obtained by fermentation of *Penicillium chrysogenum* transformant T1.48 as described in Example 4 of WO 2007/147827 (2 liter, containing 2.5 g/kg pravastatin and 0.8 g/kg compactin) was centrifuged and the supernatant was divided into 2 portions of 800 ml. One of these portions of supernatant (800 ml), ethyl acetate (800 ml) and dicalite 448 (20 g) were mixed and the pH was adjusted to 4 with 6N sulfuric acid. The mixture was filtered over a Seitz Z-2000 filter plate. The phases of the filtrate were separated and the organic phase was washed once with water (400 ml), giving an ethyl acetate extract (700 ml) which was divided into eight portions of 80 ml and one of 60 ml. Each portion was mixed with an equal volume of water at a certain pH, varying from 4.5 to 8. After mixing, the phases were separated and analyzed by HPLC. The results are given in Table 1.

**Table 1 Back extraction from ethyl acetate**

| **pH** | **Aqueous phase** | | | **Organic phase** | | | **Distribution coefficient** | | **Extraction yield** | |
|---|---|---|---|---|---|---|---|---|---|---|
| | Vol | Prava | Comp | Vol | Prava | Comp | Prava | Comp | Prava | Comp |
| | (ml) | (area) | (area) | (ml) | (area) | (area) | | | (%) | (%) |
| 4.5 | 87 | 14.0 | 0.0 | 73 | 159.9 | 16.8 | 0.1 | 0.0 | 9.4 | 0.0 |
| 5 | 90 | 32.5 | 0.0 | 72 | 142.1 | 17.0 | 0.2 | 0.0 | 22.2 | 0.0 |
| 5.5 | 88 | 52.2 | 0.0 | 72 | 120.8 | 17.2 | 0.4 | 0.0 | 34.6 | 0.0 |
| 6 | 88 | 90.0 | 0.0 | 72 | 76.2 | 16.7 | 1.2 | 0.0 | 59.1 | 0.0 |
| 6.25 | 66 | 105.3 | 0.8 | 51 | 54.9 | 16.7 | 1.9 | 0.0 | 71.3 | 5.7 |
| 6.5 | 88 | 123.4 | 1.5 | 71 | 35.2 | 15.8 | 3.5 | 0.1 | 81.3 | 10.2 |
| Comp. 7 | 89 | 138.3 | 3.6 | 71 | 13.4 | 13.1 | 10.3 | 0.3 | 92.8 | 25.6 |
| Comp. 7.5 | 90 | 144.7 | 7.1 | 70 | 5.3 | 8.8 | 27.4 | 0.8 | 97.2 | 51.0 |
| Comp. 8 | 90 | 147.1 | 11.0 | 71 | 1.6 | 3.8 | 94.1 | 2.9 | 99.2 | 78.5 |

| | | | | | | | | | | |
|---|---|---|---|---|---|---|---|---|---|---|
| Distribution coefficient is defined as C_{Aqueous phase}/C_{Organic phase}, wherein C stands for the concentration of the component in question; Prava = pravastatin; Comp = compactin. | | | | | | | | | | |

### Example 2

### Separation between pravastatin and compactin by batch-wise single stage back-extraction from methyl isobutyl ketone

One of the portions of supernatant (800 ml) obtained in Example 1, methyl isobutyl ketone (800 ml) and dicalite 448 (20 g) were mixed and the pH was adjusted to 4 with 6N sulfuric acid. The mixture was filtered over a Seitz Z-2000 filter plate. The phases of the filtrate were separated and the organic phase was washed once with water (400 ml), giving a methyl isobutyl ketone extract (750 ml) from which eight portions of 80 ml were taken. Each portion was mixed with an equal volume of water at a certain pH, varying from 4.5 to 8. After mixing, the phases were separated and analyzed by HPLC. The results are given in Table 2.

**Table 2 Back extraction from methyl isobutyl ketone**

| **pH** | **Aqueous phase** | | | **Organic phase** | | | **Distribution coefficient** | | **Extraction yield** | |
|---|---|---|---|---|---|---|---|---|---|---|
| | Vol | Prava | Comp | Vol | Prava | Comp | Prava | Comp | Prava | Comp |
| | (ml) | (area) | (area) | (ml) | (area) | (area) | | | (%) | (%) |
| 4.5 | 87 | 25.5 | 0.0 | 73 | 124.8 | 13.6 | 0.2 | 0.0 | 19.6 | 0.0 |
| 5 | 90 | 28.7 | 0.0 | 72 | 122.2 | 12.8 | 0.2 | 0.0 | 22.7 | 0.0 |
| 5.5 | 88 | 55.5 | 0.0 | 72 | 91.6 | 11.6 | 0.6 | 0.0 | 42.5 | 0.0 |
| 6 | 88 | 89.1 | 0.0 | 72 | 63.8 | 10.6 | 1.4 | 0.0 | 63.0 | 0.0 |
| 6.5 | 88 | 118.4 | 0.8 | 71 | 29.6 | 11.0 | 4.0 | 0.1 | 83.2 | 8.5 |
| Comp. 7 | 89 | 139.2 | 2.6 | 71 | 11.6 | 11.7 | 12.0 | 0.2 | 93.8 | 22.1 |
| Comp. 7.5 | 90 | 147.1 | 6.6 | 70 | 3.9 | 7.6 | 37.5 | 0.9 | 98.0 | 53.0 |
| Comp. 8 | 90 | 152.6 | 10.9 | 71 | 1.8 | 3.3 | 83.8 | 3.3 | 99.1 | 80.9 |

| | | | | | | | | | | |
|---|---|---|---|---|---|---|---|---|---|---|
| Distribution coefficient is defined as C_{Aqueous phase}/C_{Organic phase}, wherein C stands for the concentration of the component in question; Prava = pravastatin; Comp = compactin. | | | | | | | | | | |

### Example 3

### Separation between pravastatin and compactin by batch-wise multi stage cross-current back-extraction from isobutyl acetate at pH 6

Starting material for this experiment was broth filtrate, obtained after ultra-filtration of broth obtained by fermentation of *Penicillium chrysogenum* transformant T1.48 as described in Example 4 of WO 2007/147827 using a 50 nm poly-sulfone membrane. The filtrate (2 liter, containing 1.2 g/l pravastatin and 0.37 g/l compactin) was extracted twice with isobutyl acetate (2 x 1 liter) at pH 4. For a clear phase separation, the organic phases were filtered through a Seitz Z-2000 filter plate. The organic phases were combined and washed with water (500 ml), giving isobutyl acetate extract (1960 ml) which was concentrated to 100 ml under vacuum at T< 40°C. The concentrate was decolorized using a coal cartridge filled with Cuno C55 carbon (1.8 g carbon; diameter of carbon plate 18 cm²). After rinsing a clear solution (150 ml) was obtained containing ∼12 g/l pravastatin and 3.8 g/l compactin. This solution was extracted three times with water (3 x 150 ml) at pH 6. The pH was adjusted with 25% ammonia. The aqueous and organic phases were analyzed with HPLC (Table 3). As can be calculated, after the second back-extraction the total extraction yield of pravastatin in the combined aqueous phases was 97.6% pravastatin and after the third back-extraction the total extraction yield of pravastatin in the combined aqueous phases was 99.6%. The ratio pravastatin:compactin was 3:1 in the starting mixture, 45:1 after one back-extraction, 25:1 after two back-extractions and 17:1 after three back-extractions.

**Table 3 Cross-current back extraction from isobutyl acetate at pH 6**

| **Extraction** | **Aqueous phase** | | | **Organic phase** | | | **Distribution coefficient** | | **Extraction yield** | |
|---|---|---|---|---|---|---|---|---|---|---|
| | Vol | Prava | Comp | Vol | Prava | Comp | Prava | Comp | Prava | Comp |
| | (ml) | (area) | (area) | (ml) | (area) | (area) | | | (%) | (%) |
| 1^{st} | 150 | 188.6 | 4.2 | 150 | 39.1 | 67.8 | 4.8 | 0.06 | 82.8 | 5.8 |
| 2^{nd} | 155 | 40.3 | 4.9 | 145 | 6.8 | 70.2 | 5.9 | 0.07 | 86.3 | 7.0 |
| 3^{rd} | 150 | 6.5 | 4.7 | 145 | 1.2 | 67.6 | 5.2 | 0.07 | 84.4 | 6.8 |

| | | | | | | | | | | |
|---|---|---|---|---|---|---|---|---|---|---|
| Distribution coefficient is defined as C_{Aqueous phase}/C_{organic phase}, wherein C stands for the concentration of the component in question; Prava = pravastatin; Comp = compactin. | | | | | | | | | | |

### Example 4

### Separation between pravastatin and compactin by batch-wise multi stage cross-current back-extraction from ethyl acetate at pH 5.9

Starting material for this experiment was broth filtrate, obtained after ultra-filtration of broth obtained by fermentation of *Penicillium chrysogenum* transformant T1.48 as described in Example 4 of WO 2007/147827 using a 50 nm poly-sulfone membrane. The filtrate (5.25 liter, containing 1.2 g/l pravastatin and 0.37 g/l compactin) was extracted twice with ethyl acetate (2 x 2.5 liter) at pH 4. For a clear phase separation, the organic phases were filtered through a Seitz Z-2000 filter plate. The extraction yields were 90.6% and 8.3 % respectively, so the overall extraction yield was 98.9 %.
The organic phases were combined and washed with water (2.5 liter), giving ethyl acetate extract (4.65 liter, yield over the wash step 97%) which was concentrated to 250 ml under vacuum at T< 40°C, giving a clear solution containing -25 g/l pravastatin. This solution was extracted three times with water (3 x 250 ml) at pH 5.9. The pH was adjusted with 4N sodium hydroxide. The aqueous and organic phases were analyzed with HPLC (Table 4). As can be calculated, after the second back-extraction the total extraction yield of pravastatin in the combined aqueous phases was 91% pravastatin and after the third back-extraction the total extraction yield of pravastatin in the combined aqueous phases was 96%. The ratio pravastatin:compactin was 3:1 in the starting mixture, 26:1 after one back-extraction, 19:1 after two back-extractions and 17:1 after three back-extractions.

**Table 4 Cross-current back extraction from ethyl acetate at pH 5.9**

| **Extraction** | **Aqueous phase** | | | **Organic phase** | | | **Distribution coefficient** | | **Extraction yield** |
|---|---|---|---|---|---|---|---|---|---|
| | Vol | Prava | Comp | Vol | Prava | Comp | Prava | Comp | Prava |
| | (ml) | (area) | (area) | (ml) | (area) | (area) | | | (%) |
| 1^{st} | 280 | 51.3 | 2.0 | 225 | 34.2 | 26.5 | 1.5 | 0.08 | 65.1 |
| 2^{nd} | 280 | 20.7 | 1.7 | 195 | 11.0 | 27.5 | 1.9 | 0.06 | 73.0 |
| 3^{rd} | 275 | 4.5 | 0.8 | 165 | 6.4 | 31.1 | 0.7 | 0.03 | 53.9 |

| | | | | | | | | | |
|---|---|---|---|---|---|---|---|---|---|
| Distribution coefficient is defined as C_{Aqueous phase}/C_{Organic phase}, wherein C stands for the concentration of the component in question; Prava = pravastatin; Comp = compactin. | | | | | | | | | |

## Claims

1. A method for the purification of pravastatin comprising extracting a solution comprising pravastatin in a water-immiscible solvent with water or an aqueous solution **characterized in that** said extraction is carried out at a pH value ranging from 5.0 to 6.5 and that said water-immiscible solvent is an acetate or a ketone.

2. Method according to claim 1 further comprising isolating pravastatin from the aqueous phase obtained after said extracting.

3. Method according to any one of claims 1 to 2 wherein said pH value is ranging from 5.5 to 6.5.

4. Method according to any one of claims 1 to 3 wherein said acetate is chosen from the list consisting of ethyl acetate, iso-butyl acetate, methyl acetate and propyl acetate and said ketone is methyl iso-butyl ketone.

5. Method according to any one of claims 1 to 4 wherein said extraction is carried out in a counter-current mode or in a cross-current mode.

## Patentansprüche

1. Verfahren zur Reinigung von Pravastatin, bei dem man eine Pravastatin enthaltende Lösung in einem mit Wasser nicht mischbaren Lösungsmittel mit Wasser oder einer wässrigen Lösung extrahiert, **dadurch gekennzeichnet, dass** man die Extraktion bei einem pH-Wert im Bereich von 5,0 bis 6,5 durchführt und es sich bei dem mit Wasser nicht mischbaren Lösungsmittel um einen Essigsäureester oder ein Keton handelt.

2. Verfahren nach Anspruch 1, bei dem man ferner Pravastatin aus der nach dem Extrahieren erhaltenen wässrigen Phase isoliert.

3. Verfahren nach einem der Ansprüche 1 bis 2, bei dem der pH-Wert im Bereich von 5,5 bis 6,5 liegt.

4. Verfahren nach einem der Ansprüche 1 bis 3, bei dem man den Essigsäureester aus der Liste bestehend aus Essigsäureethylester, Essigsäureisobutylester, Essigsäuremethylester und Essigsäurepropylester auswählt und es sich bei dem Keton um Methylisobutylketon handelt.

5. Verfahren nach einem der Ansprüche 1 bis 4, bei dem man die Extraktion in Gegenstromfahrweise oder Querstromfahrweise durchführt.

## Revendications

1. Procédé pour la purification de pravastatine comprenant l'extraction d'une solution comprenant de la pravastatine dans un solvant non miscible avec l'eau avec de l'eau ou une solution aqueuse **caractérisé en ce que** ladite extraction est effectuée à une valeur de pH allant de 5,0 à 6,5 et **en ce que** ledit solvant non miscible avec l'eau est un acétate ou une cétone.

2. Procédé selon la revendication 1 comprenant en outre la séparation de la pravastatine de la phase aqueuse obtenue après ladite extraction.

3. Procédé selon l'une quelconque des revendications 1 à 2 dans lequel ladite valeur de pH va de 5,5 à 6,5.

4. Procédé selon l'une quelconque des revendications 1 à 3 dans lequel ledit acétate est choisi parmi l'acétate d'éthyle, l'acétate d'isobutyle, l'acétate de méthyle et l'acétate de propyle et ladite cétone est la méthylisobutylcétone.

5. Procédé selon l'une quelconque des revendications 1 à 4 dans lequel ladite extraction est effectuée en un mode à contre-courant ou en un mode à courant transversal.
